# EUROPEAN PATENT APPLICATION

(11) **EP 3 888 699 A1**
(43) Date of publication of application: **06.10.2021**
(21) Application number: 20175494.2
(22) Date of filing: 19.05.2020
(51) Int. Cl.: A61L 2/12, A61L 9/18

(54) **VIRUS INACTIVATION IN FLOWING FLUIDS**

(30) Priority: 02.04.2020 TR 202005289
(71) Applicant: SMARTE TEKNOLOJI VE ENERJI SANAYI TICARET A.S., 34467 Istanbul (TR); PLUSSMARTE DANMARK APS, 1220 København K (DK); Ballikaya, Melih, 34467 Istanbul (TR)
(72) Inventor: BALLIKAYA, Melih, 34467 Istanbul (TR)
(74) Representative: Sevinç, Erkan

(57) **Abstract**

The present invention proposes an apparatus (1) which includes a block (20) having a first side wall (21) and a second side wall (22) opposing said first side wall (21), arranged for allowing flow of fluids in an intended fluid flow direction (D) transverse to a plane passing through both the first side wall (21) and second side wall (22), and one or more microwave emitting connector(s) (11) arranged for provision of the block (20) with microwaves transverse to said intended fluid flow direction (D).

## Description

### Technical field of the invention

The present invention relates to an apparatus for inactivating viruses in flowing fluids. In particular, the present invention relates to inactivation of viruses using microwave.

### Background of the Invention

Disinfection by inactivation of viruses mainly includes the use of ultraviolet (UV) radiation, or chemical agents such as oxidants.

For inactivation of viruses in flowing fluids with the principles of chemistry, chemical agents inevitably mix to said fluid and get dragged along with the stream. Thus, the fluid gets contaminated. In the cases where the fluid is air or water to be directly contacted with mammals such as humans, the chemical agents should be separated from the fluid for elimination of possible harm. Such separation process brings extra and unmanageable costs. Disinfection by chemical agents take a high amount of time (e.g. around 150 minutes), and cannot be completed whilst the passage of a fluid through an apparatus dedicated to such disinfection without necessitating a long residence time. Hence, chemical means are not sufficiently suitable for inactivating viruses in flowing streams of fluids.

On the other hand, UV radiation itself is normally insufficient for an effective inactivation of viruses in flowing fluids, unless a tremendous amount of energy is consumed in the process. When the fluid is air, UV radiation causes the formation of ozone, which is harmful for living bodies.

WO 2004/069288 A2 discloses the use microwaves as an alternative to UV radiation in sterilization of air. Air is passed through a wire mesh of a sterilization chamber. A vapor or a gas is adhered onto microorganisms to form a complex structure, and then said complex structure is subjected to high energy microwaves or UV radiation to cause a high electrostatic load on the complex structure, resulting in disinfection. Apparently, heating of the complex structure is also an important part of the related disinfection process.

US 2004/ 120 845 A1 relates to removal of pathogens in air circulated by HVAC systems. The system employs an ozone generator for preparing ozone to be mixed with water to obtain an oxidizing chemical agent. UV radiation is used in the formation of ozone from air oxygen. Microwaves are discussed as an alternative to UV radiation. Accordingly, said publication uses microwaves in production of ozone as a step in process for obtaining the oxidizing chemical agent.

It is assumed that in an indoors space, airborne viruses endure for days. In the case where the indoors space is air conditioned, the viruses are easily distributed all around the space, which aggravates a spread. Thus, in the process of normalization after a pandemic such as COVID-19, use of air conditioning systems in public indoor spaces (such as restaurants, workspaces or shopping malls) must be avoided, because air streaming from the air conditioning systems expedites the spread of the infection. Therefore, all of the advantages related to air conditioning shall remain unavailable until the pandemic is completely over.

Hence, inactivation of viruses in flowing fluids such as air or water is still an unsolved concern. This is especially the case when it comes to high fluid flow rates such as those air conditioning or ventilation systems, or systems for circulating swimming pools.

### Objects of the Invention

The primary object of the present invention is to overcome the drawbacks outlined above.

Another object of the present invention is to propose an apparatus to be used in inactivation of viruses in streaming fluids.

A further object of the present invention is to propose an apparatus which is also easy to produce with low cost, which is easy to operate, which has long service life and easy to maintain by having a simple structure.

Other objects of the present invention will become apparent from accompanied drawings, brief descriptions of which follow in the next section as well as appended claims.

### Summary of the Invention

The apparatus proposed in the present application has a simple structure, which is easy to produce with low cost. The simple structure is also durable for a long service life, and easy to maintain. The apparatus does not consume high amounts of energy when in use, and is therefore economically advantageous in terms of operational costs. The disinfection process does not result in contamination of the processed fluid with harmful chemicals such as organic disinfectants or ozone.

The apparatus according to the present invention includes a block having a first side wall and a second side wall. The first side wall and second side wall oppose each other. The block is arranged for allowing flow of fluids in an intended fluid flow direction transverse to a plane passing through both the first side wall and second side wall. The apparatus further comprises one or more microwave emitting connectors arranged for provision of the block with microwaves transverse to said intended fluid flow direction (D).

### Brief Descriptions of the Drawings

The appended drawings, brief description of which are provided below, are given solely for the purpose of exemplifying embodiments according to the present invention.
Fig.1 shows a schematic plan view of an exemplary embodiment of the apparatus according to the present invention, along an intended fluid flow direction.
Fig.2 shows a schematic plan view of another exemplary embodiment of the apparatus according to the present invention.
Fig.3(a) schematically shows another exemplary embodiment of the apparatus according to the present invention.
Fig.3(b) schematically shows another exemplary embodiment of the apparatus according to the present invention.
Fig.4(a) is a perspective view of an exemplary embodiment of the block, which is broad.
Fig.4(b) shows perspective view of a plurality of microwave reflectors (23) for being provided inside a block as shown in Fig.4(a).
Fig.4(c) schematizes an exemplary provision of the microwave reflectors (23) of Fig.4(b) into the block of Fig.4(a).
Fig.5(a) is perspective view of a block with microwave reflectors (23).
Fig.5(b) is a plan view of the block shown in Fig.5(a).
Fig.6 shows an exploded view of a block assembly which includes perforated plates (30) sandwiching the block.
Fig.7(a) is perspective view of a block assembly as referred to in Fig.6, in which the block is provided with one or more microwave emitting connector(s).
Fig.7(b) is perspective view of a block assembly as referred to in Fig.6, in which the block is provided with one or more microwave emitting connector(s) and corresponding one or more microwave receiving connector(s).
Fig.7(c) is perspective view of a block assembly as referred to in Fig.6, in which the block corresponds to an embodiment as referred to in Fig.5(a) and Fig.5(b).
Fig.8(a) shows exploded view in preparation of an exemplary alignment of the block (or block assembly) with a fluid flow port.
Fig.8(b) shows an exploded view comparable to that which is shown in Fig.8(a).
Fig.9(a) is schematic view of an exemplary apparatus including a block showing an RF signal generator for producing relevant signals to be fed to the RF power source supplying energy to the microwave emitting connector.
Fig.9(b) is schematic view of an exemplary apparatus including a block showing a RF signal generator for producing relevant signals to be fed to the RF power source supplying RF energy to the microwave emitting connector.
Fig.9(c) is schematic view of an exemplary apparatus as in the Fig.9(b), showing a multiplicity of further optional and preferred features.

### Detailed Description of the Invention

Hereinafter, preferred embodiments of the present invention will be described in detail with reference to the accompanying drawings.

The present specification proposes an apparatus which effectively disinfect flowing fluid streams by inactivating viruses therein. The apparatus makes use of acoustic vibration, thereby causing resonance which mechanically damages outmost layers of viruses. Although the apparatus is effective in a wide variety of viruses (such as various types of Ebola, influenza, SARS, etc.) by easily arranging the frequency and energy density levels in accordance with a respective virus; the inactivation of spherical envelops is particularly easy due to their geometric limitations in terms of envelope surface area capable of holding the genetic material and enzymes in their inner volume. Thus, the inactivation occurs instantly (i.e. within a very short residence time) and with consumption of a very low amount of energy. In particular, disintegration of envelopes requires a minimum amount of energy and time, in inactivation of lipid enveloped viruses such as coronaviruses. Yet, advantages of the apparatus are not to be limited only to those related to coronaviruses, and it is possible to inactivate other viruses and even microorganisms which require a higher extent of acoustic vibration in disintegration thereof.

Within the context of the present application, the apparatus (1) applies acoustic resonance to be applied onto viruses in a fluid stream whilst passing through a block (20).

Within the context of the present application, the term "microwave" refers to that with a frequency to induce acoustic vibration on an outermost layer of viruses, causing mechanical damage of such outermost layer. It is observed that virus species can be destroyed in this way, by defining and selecting microwave frequency ranges specific thereto. For instance, it is observed that frequencies within the range between 8.0 GHz and 9.5 GHz cause momentary deformations on envelopes of SARS-COV-2, resulting in rupture of said envelopes. Therefore, this range is considered suitable for being employed in damaging coronaviruses. When the frequency is within the narrower range between 8.2 GHz and 8.8 GHz, the efficiency in inactivation of SARS-COV-2 further increases, and a frequency around 8.4 GHz (i.e. within the range between 8.3 GHz and 8.5 GHz) is considered sweet-spot in inactivation of such viruses in lowest time and with lowest energy consumption.

Since the present invention relies on mechanically damaging viruses by acoustic resonance, the apparatus (1) practically does not necessitate nor cause any heating of the fluid. Considering that heating would denature further biochemical/biological structures other than viruses, the apparatus (1) according to the present invention also enables a safe and effective sterilization of liquids such as vaccines, or bodily fluids such as blood samples. Therefore, the apparatus (1) according to the present invention and method of virus inactivation by using such apparatus is not to be limited for use in air or water disinfection, but also to in-vitro sterilization of other fluids with delicate organic or biological material which should be maintained, such as vaccines, blood samples, spinal fluid samples, etc.

The apparatus (1) includes a block (20) (disinfection block) having a first side wall (21) and a second side wall (22) opposing said first side wall (21), arranged for allowing flow of fluids in an intended fluid flow direction (D) transverse to a plane passing through both the first side wall (21) and second side wall (22). The apparatus (1) further includes one or more microwave emitting connector(s) (11) arranged for provision of the block (20) with microwaves transverse to said intended fluid flow direction (D).

The microwave with a designated frequency can be thus applied into the block (20) via said one or more microwave emitting connector(s) (11), thereby causing acoustic resonance on the outermost layer of viruses in the passing fluid. For instance, in SARS-COV-2, the momentary envelope deformations acoustic resonance causes deviations from its original diameter (to extents calculable to be around 30 nm), resulting in inactivation due to mechanical damage in envelope structure. The same applies to other virus species by selection and easily replacing the one or more microwave emitting connector(s) (11) in accordance with another, frequency specific to another virus. Considering different sizes and outmost layer structures, the same principle applies to virus species other than SARS-COV-2, by employing respective specific frequencies selected from a range between 1 GHz and 20 GHz. Considering that currently SARS-COV-2 is the most urgent issue of the world, the frequency is preferably to be kept within the range between 8.0 GHz and 9.5 GHz, more preferably between 8.2 GHz and 8.8 GHz, even more preferably between 8.3 GHz and 8.5 GHz. Considering that commercially available and low cost waveguides (such as WR90) can be used as microwave emitting connectors (11), the frequency can be arranged to be within the range between 6.5 GHz and 13 GHz which provides a single-mode provision of adjustable frequency, and within the range between 7 GHz and 12 GHz for an even higher accuracy. In rod-shaped (i.e. substantially cylindrical) viruses, 5.5-7 GHz provides a peak in resonance, and 20-25 GHz, 33-37 GHz and 45-50 GHz are also effective in inactivation of such viruses. On the other hand, a frequency range within 6-10 GHz is considered as an intersection applicable to inactivation of both spherical viruses and rod-shaped viruses. Hence, the apparatus (1) can be easily adapted in accordance with a specific frequency designated to inactivation of a target virus.

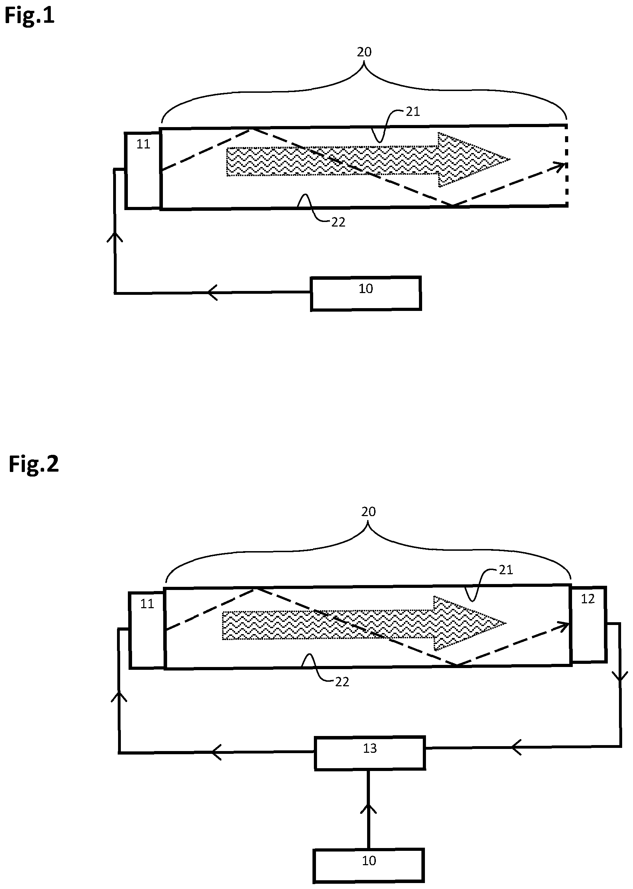

The block (20) can be considered as a frame allow fluid passage therethrough. The block (20) can be in the form of an open frame such as it is visually exemplified in Fig.1. Yet the block (20) is preferably in the form of a closed frame such as it is visually exemplified in the rest of the attached drawings. The form of closed frame enables confinement of a fluid stream therethrough, thus eliminates by-pass streams and enhances the effectiveness in fluid disinfection.

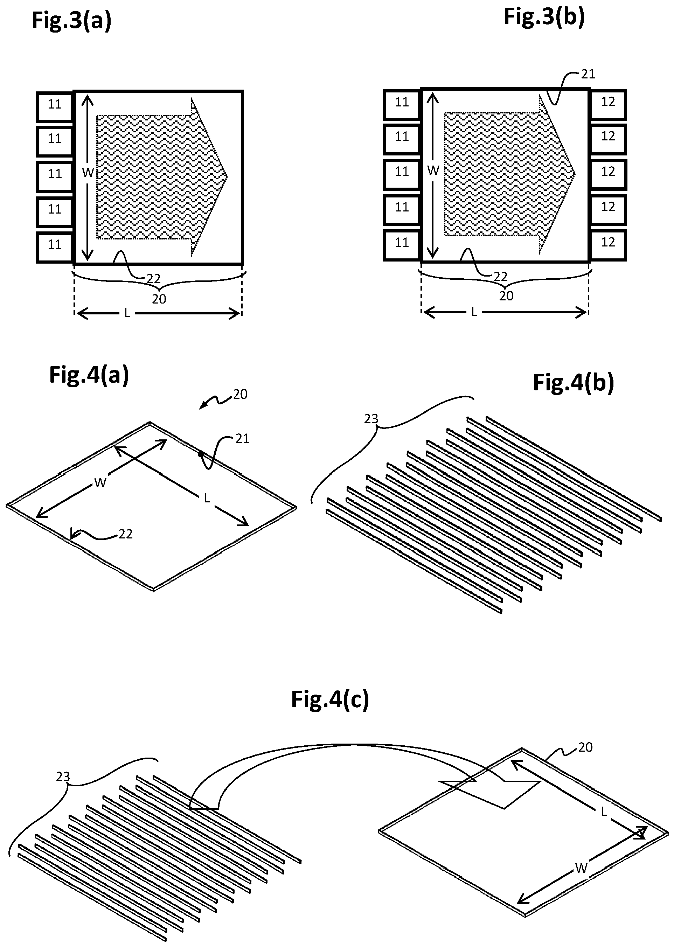

Fig.1 shows a schematic plan view of an exemplary embodiment of the apparatus (1) according to the present invention, along an intended fluid flow direction. Thus the intended fluid flow direction corresponds to a viewer's aspect direction transverse or orthogonal to the drawing plane, therefore cannot be shown in this drawing; and the same applies to Fig.2, Fig.3(a), Fig.3(b), Fig.5(b), Fig.9(a), Fig.9(b) and Fig.9(c). An exemplary general trajectory of microwave beams released from the microwave emitting connector (11) into the block (20) is represented using a shaded bold arrow along the block (20) (the same also applies to Fig.2, Fig.3(a) and Fig.3(b)). Here a microwave beam and its possible reflections from a first side wall (21) and a second side wall (22) of the block (20), are depicted using dashed lines.

Fig.2 shows a schematic plan view of another exemplary embodiment of the apparatus (1) according to the present invention. Here, the apparatus (1) is preferably provided with a microwave receiving connector (12), which is arranged to receive/collect microwave beams released from the microwave emitting connector (11) into the block (20), e.g. upon such microwave beams travelling through the block (20). Thus, microwaves travelled along the block (20) are collected by the microwave receiving connector (12). The apparatus (1) can be provided with a plurality of such microwave emitting connectors (11) and such microwave receiving connectors (12). The microwaves (i.e. their remaining energy) collected by the microwave receiving connector(s) (12) can be conducted to one or more circulator(s) (13) (within the present context, one or more RF circulator(s) or microwave circulator(s)) arranged to further conduct/feed the collected microwaves to the microwave emitting connector(s) (11). Accordingly, the apparatus (1) can be further provided with one or more circulator(s) (13) for at least partial recovery of the microwave energy from the microwave receiving connector (12) to supply the microwave emitting connector (11). A difference between microwave energy extents at the microwave emitting connector (11) and the microwave receiving connector (12) can be replenished by one or more power source(s) (10) (within the present context, one or more RF power source(s) or microwave power source(s)) in communication with the microwave circulator.

Fig.3(a) schematically shows another exemplary embodiment of the apparatus (1) according to the present invention, provided with a plurality of microwave emitting connectors (11) at a microwave introduction side of the block (20), which are arranged for provision of a rather uniform distribution of microwaves from said microwave introduction side throughout the block (20) and across an intended fluid flow direction substantially orthogonal to the general trajectory of microwaves. The microwave emitting connectors (11) being plural is especially useful where the ratio between a distance between a first side wall (21) and a second side wall (22) for defining a width (W) of the block (20) to be scanned by the microwaves, to a length (L) of the block (20) to be scanned by the microwaves is higher than 0.2, since such block (20) is considered "broad" within the context of the present application. The multiplication product of said width (W) and length (L) can be considered to correspond an area to be scanned by microwaves within the block (20), and across the intended fluid flow direction.

Fig.3(b) schematically shows another exemplary embodiment of the apparatus (1) according to the present invention. Referring to the advantage of the apparatus (1) depicted in Fig.2, this embodiment differs from that shown in Fig.3(a) in that a plurality of microwave receiving connectors (12) are provided another side of the block (20) opposing the microwave beams from the microwave emitting connectors (11). Thus, the plurality of microwave receiving connectors (12) are arranged to collect the microwaves released from the microwave emitting connectors (11) upon travelling along said area to be scanned. Upon receiving said collected microwaves, the same can be then circulated to said plurality microwave emitting connectors (11) through respective circulator(s). Any difference between microwave energy extents at the microwave emitting connectors (11) and the microwave receiving connectors (12) can be replenished by microwave power source(s) in communication with the microwave circulator(s).

In a preferred embodiment, the apparatus (1) is provided with one or more reflectors (23) separating the block (20) into two or more corridors (C) for meandering of microwaves. The reflectors (23) can be considered as further walls able to reflect microwaves transverse the intended fluid flow direction (D), e.g. by being formed from a microwave reflecting material such as metals, e.g. tin or aluminum. With such meandering of microwaves, the extent of scanning inside the block (20) is increased or can be maintained even with a minimal number of microwave emitting connectors (11), notwithstanding the length (L), width (W) or ratio therebetween. Thus, an effective microwave scanning/sweeping (for disinfection) of an inner space of the block (20) can be effected with e.g. a single microwave emitting connector (11). This advantage further allows an effective collection of microwaves upon scanning the block (20) at the end of a single corridor (C). Thus, such embodiment also renders a minimal number of microwave receiving connectors (12) sufficient for effective recycling of microwaves; and even a single microwave receiving connector (12) can suffice. Furthermore, with such embodiment which allows meandering, the block (20) can be provided with a minimal number of the microwave emitting connector(s) (11) and microwave receiving connector(s) (12) (even a single one of each) at a single side in common.

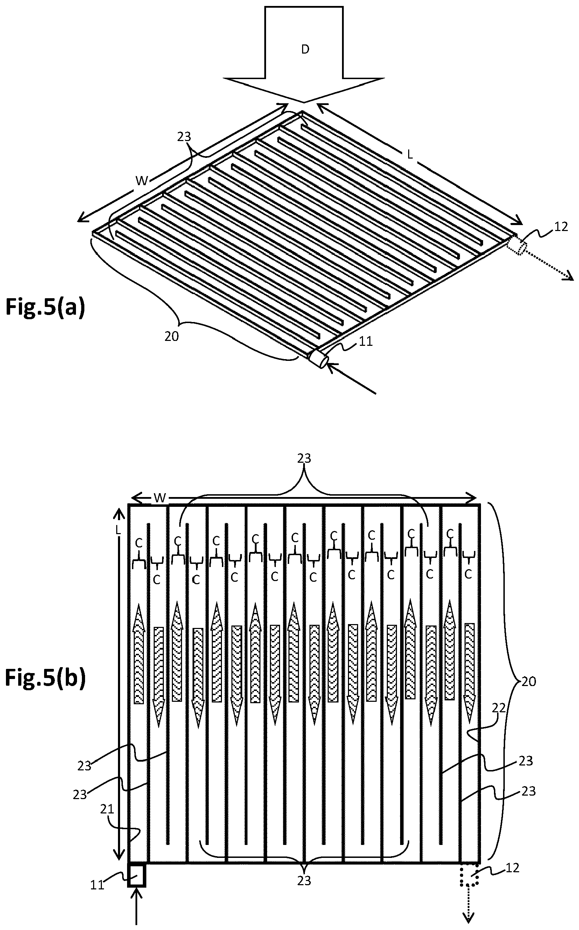

Fig.4(a) is a perspective view of an exemplary embodiment of the block (20), which is broad; i.e. the ratio between a distance between a first side wall (21) and a second side wall (22) for defining a width (W) of the block (20) to be scanned by the microwaves, to a length (L) of the block (20) to be scanned by the microwaves is higher than 0.2. Fig.4(b) shows perspective view of a plurality of microwave reflectors (23) for being provided inside a block (20) as shown in Fig.4(a). Fig.4(c) schematizes an exemplary provision of the microwave reflectors (23) of Fig.4(b) into the block (20) of Fig.4(a). Fig.5(a) is perspective view of a block (20) provided with a plurality of microwave reflectors (23). Fig.5(b) is a plan view of the block (20) shown in Fig.5(a).

As represented in Fig.5(b) using respective shaded arrows, a general trajectory of microwaves from a microwave emitting connector (11) can be confined within a narrowed corridor (C) by means of the reflecting effect available by the presence of the microwave reflectors (23). The number of the microwave reflectors (23) can be arranged in accordance with the number of microwave emitting connectors (11), to enhance the efficiency of scanning a fluid across its intended flow direction even with a reduced number of microwave emitting connectors (11). For instance, an effective scanning can be achieved by using even a single microwave emitting connector (11) as shown in Fig.5(a) and Fig.5(b). The optional (yet preferred) presence of microwave receiving connector(s) (12) is symbolized over dotted depiction, and a reduced number of microwave receiving connector(s) (12) (even a single one) is sufficient for effectively collecting of the microwaves from the block (20) for the above-mentioned circulation/recycling/reuse. General trajectories of microwaves travelling along corridors (C) are symbolized using shaded arrows, yet it is clear that this does not mean that all of microwave beams are parallel to each other. Instead, in this embodiment it is preferred that the one or more microwave emitting connectors (11) are arranged to scatter microwaves. Thereby, to achieve a more uniform meandering of microwaves throughout the block (20), an enhanced extent of reflections of the microwaves is effected:
- along an entry corridor (C) (i.e. between a first side wall (21) and an opposing microwave reflector (23)),
- then along one or more intermediate corridors (C) (i.e. between two consecutive microwave reflectors (23) if more than one reflectors (23) are used), and then
- along an exit corridor (C) (i.e. between a first side wall (21) and an opposing microwave reflector (23)).

For any of the embodiment of the blocks (20) according to the present invention, the one or more microwave emitting connectors (11) can be arranged to scatter microwaves on a scanned plane which is transverse (preferably orthogonal) to the intended fluid flow direction (D), e.g. by emitting the microwaves through one or more slits (e.g. waveguides) along a direction on the scanned plane. Alternatively, or furthermore, the one or more microwave emitting connectors (11) are provided with a motor-guided microwave directing means to allow arrangement (i.e. setting and/or manipulation and/or alternation) of the directions of microwaves (e.g. beams thereof). The motor-guided microwave directing means can be arranged for emitting non-scattering microwave beams parallel to each other, and can be further arranged to achieve an acute-angled direction of microwave beams towards a first side wall (21) or where applicable, towards a microwave reflector (23). Thus, selection of an angular sweet-spot is enabled to maximize the scanning efficiency in accordance with the meandering of the microwave beams throughout the block (20).

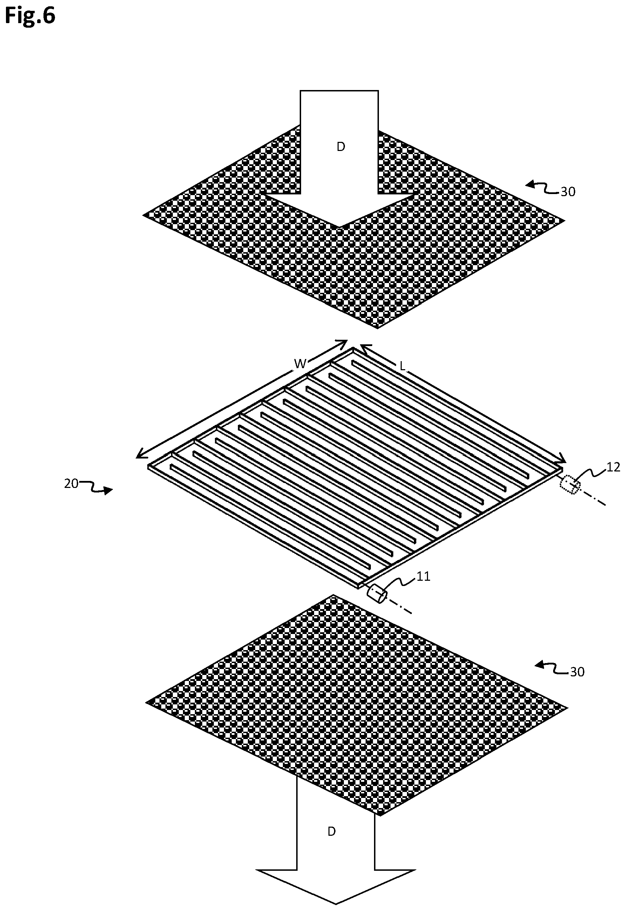

In a preferred embodiment, the apparatus (1) can include perforated plates (30) sandwiching a block (20). Fig.6 shows an exploded view of a block (20) assembly which includes perforated plates (30) sandwiching the block (20). Perforations on the perforated plates (30) allow fluid flow in the intended fluid flow direction (represented using bold white arrow). The perforated plates (30) are different from a wire mesh in that they inherently include a substantial extent of surface opposing the block (20) (i.e. said surface is substantially orthogonal to the intended fluid flow direction); and this feature confines microwaves mainly within the block (20), simultaneously allowing a stream of fluid to be disinfected. In other words, the perforated plates (30) are arranged for minimization of any possible escape of microwaves away from the block (20) in directions having a component parallel to the intended fluid flow direction. The advantages attributed to perforated plates (30) are not limited to the block (20) embodiment shown in Fig.6, but apply to any embodiment of block (20) assemblies exemplified throughout the present specification, and to their obvious further variations.

As a further advantage, the fluid passing the perforations would inevitably create eddy streams and mix well. This effect further enhances the uniformity in virus disinfection, because such movements in the fluid causes an enhanced mixing, resulting in a uniform exertion of electromagnetic field throughout the fluid passing through the block (20).

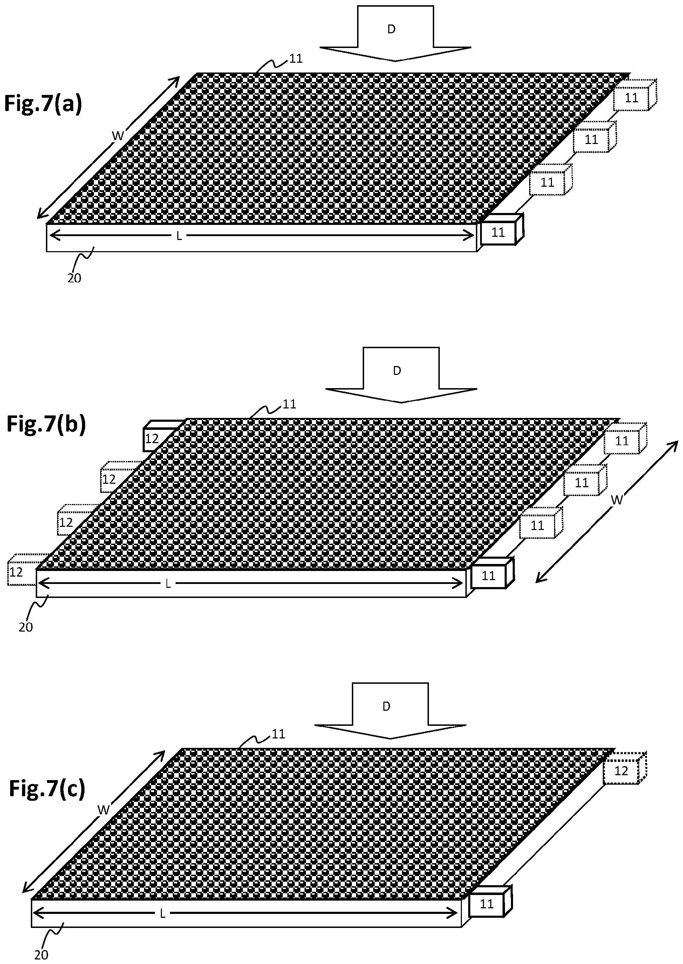

Fig.7(a) is perspective view of a block (20) assembly as referred to in Fig.6, in which the block (20) is provided with one or more microwave emitting connector(s) (11). Fig.7(b) is perspective view of a block (20) assembly as referred to in Fig.6, in which the block (20) is provided with one or more microwave emitting connector(s) (11) and corresponding one or more microwave receiving connector(s) (12), preferably arranged for re-circulation of the received microwaves to respective microwave emitting connector(s) (11). Fig.7(c) is perspective view of a block (20) assembly as referred to in Fig.6, in which the block (20) corresponds to an embodiment as referred to in Fig.5(a) and Fig.5(b). Here, the block (20) preferably further includes a microwave receiving connector (12) which is further preferably arranged for re-circulation of the received microwaves to the microwave emitting connector (11) as explained with reference to Fig.5(a) and Fig.5(b).

The fluid can be in the form of a gas such as air, or liquid such as water. For instance, the apparatus (1) can be considered to be arranged for engaging with a fluid flow port (60), thereby being arranged for forming a fluid passage to a fluid when streaming through such port (60). In an exemplary case where the fluid is a gas, e.g. air, the port (60) can be a vent in fluid communication with a pressurizing means (61) such as a fan of an air conditioning or air purification device. In another exemplary case where the fluid is a liquid, e.g. water, the port (60) can be a flow opening in fluid communication with a pressurizing means (61) such as a pump.

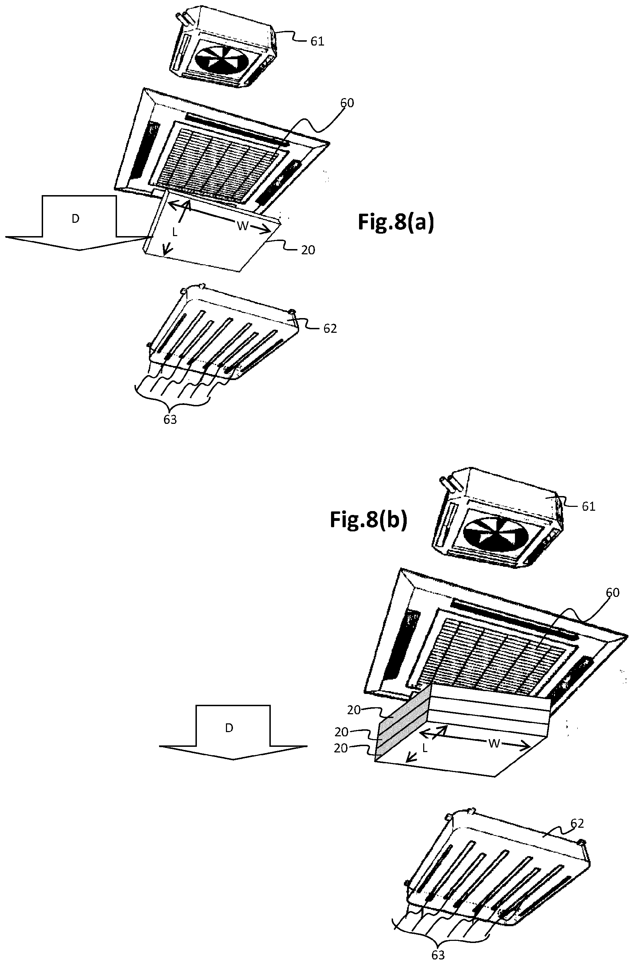

Fig.8(a) shows exploded view in preparation of an exemplary alignment of the block (20) (or block (20) assembly) with a fluid flow port (60). Fig.8(b) shows an exploded view comparable to that which is shown in Fig.8(a). Here, it is emphasized that a plurality of blocks (20) can be employed within the context of the present application. Thus, a preferred embodiment of the apparatus (1) according to the present invention further comprises a holding means (62) with fluid passage openings (63), the holding means having a size and shape to receive said one or more blocks (20), and preferably to attach said one or more blocks (20) to the port (60).

Preferably, such plurality of blocks (20) is arranged in series; i.e. such that the fluid flows/passes through the plurality of consecutively arranged blocks (20). Preferably, the plurality of blocks (20) is confined between perforated plates (30) arranged to allow said flow of the fluid through the blocks (20). This measure at least partly blocks the escape of microwave away from the plurality of blocks (20). Even more preferably, perforated plates (30) are sandwiched between consecutive blocks (20). Even more preferably, each block (20) is confined between perforated plates (30), such that a perforated plate (30) is sandwiched between each pair of consecutive blocks (20) throughout the intended flow direction. This measure enhances the uniformity in distribution of microwaves throughout the plurality of blocks (20), and enhances the accuracy and precision in disinfection. In Fig.8(a) and Fig.8(b) an exemplary way of how the fluid flow port can be in fluid communication with a propelling means is depicted, to effect the flow of a respective fluid in a fluid flow direction transverse to an area to be scanned which may be defined by the width (W) and length (L) of the block (20).

In the case where the streaming fluid is in the form of liquid such as water, it is preferred to provide the stream with gas bubbles, e.g. infuse air bubbles. This measure enhances the distribution of microwave beams inside the stream. Accordingly, for treating liquids, the apparatus (1) can include a means for provision of gas bubbles into the liquid.

In the case where the streaming fluid is air, when in use, the block (20) can be named as a "nanoacoustic airflow cell".

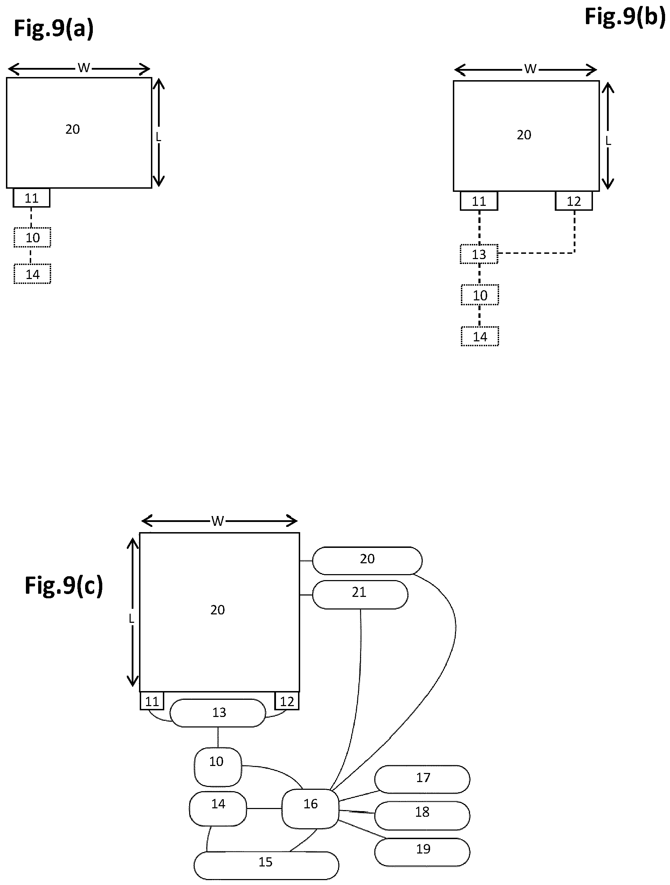

Fig.9(a) is schematic view of an exemplary apparatus (1) including a block (20) showing a RF (e.g. microwave) signal generator for producing relevant signals to be fed to the RF (e.g. microwave) power source supplying RF (microwave) energy to the microwave emitting connector (11). Although not shown, a plurality of microwave emitting connectors (11) can be used as disclosed above. In such case, the microwave emitting connectors (11) can be provided with energy by a respective number of such power sources, or can be in communication with a single power source in common.

Fig.9(b) is schematic view of an exemplary apparatus (1) including a block (20) showing a RF (e.g. microwave) signal generator for producing relevant signals to be fed to the RF (e.g. microwave) power source supplying RF (microwave) energy to the microwave emitting connector (11). Here, the apparatus (1) also includes one or more microwave receiving connectors (12) as described above, and the signal generator is in communication with a microwave circulator as discussed above, to supply a minimized extent of signals which can correspond to the extent of a difference between the emitted and received amounts at the microwave emitting connector (11) and microwave receiving connectors (12), respectively. Although not shown, a plurality of microwave emitting connectors (11) and microwave receiving connectors (12) can be used as disclosed above. In such case, the microwave emitting connectors (11) can be provided with energy by a respective number of such microwave circulators, or can be in communication with a single microwave circulator in common.

Fig.9(c) is schematic view of an exemplary apparatus (1) as in the Fig.9(b), showing a multiplicity of further optional and preferred features, any working combinations thereof apparent to a skilled person being applicable. Said optional and preferred features include a power supply (15) for fulfillment of power requirements of the apparatus (1); a data management means (16) such as a board or a computer in communication with a signal generator (14) (RF or microwave signals generator, thus within the context of the present application), with the power source (10), with a communication interface (17), and preferably with one or more of the list of the following:
- one or more fluid quality sensors (18); in the case where the fluid is air, the fluid quality sensor can be arranged to detect the status related to or more of the following: airborne particles (e.g. PM2.5), volatile organic compounds (VOCs), carbon dioxide;
- one or more presence sensors (19), i.e. sensors for detecting the presence of moving bodies such as human beings, e.g. LDR sensor, time of flight sensor, camera (e.g. thermal camera or optical camera);
- one or more probes (20) for provision of feedback about a local frequency and power status inside the block (20) (20);
- one or more further probes (21) for provision of feedback about a local temperature, and in the case where the fluid is air, for provision of feedback about local humidity inside the block (20) (20).

It is observed that a preferred extent and character in electric field is achieved when a signal from the signal generator (14) is split using a signal splitter (not shown).

Considering that the fluid to be treated with the apparatus (1) can be air, the present invention not only enables the use of HVAC systems safely, the possibility of infection in air conditioned indoor spaces is minimized by the inactivation effect of the apparatus (1). This can be very easily done by simply aligning/attaching the apparatus (1) to a vent of a HVAC system which can even be already installed.

### EXAMPLE:

A computer-simulated experimental run was conducted using a block (20) according to the present invention. The following information shows the proof-of-concept, without aiming to unduly limit the scope of the present application to disclosed specific parameter values.

The block (20) used in the experimental run had a width (W) of 10cm and a length (L) of 10cm (and a height of 1 cm, which was the orthogonal distance between perforated plates). The body (20) was provided with a plurality (four, not limiting) of reflectors (23), further provided with two perforated plates (30) sandwiching the block (20), the fluid was air. The perforations on the perforated plates (30) were circular with 2 mm diameters (not limiting), and total area of the perforations corresponded to 8% of the area to be scanned (i.e. multiplication product of width (W) and length (L)) (not limiting). The loci of perforations on a perforated plate (30) to be brought into fluid communication with a circular fan were delimited to a circular projection of a vent od said fan, instead of being distributed throughout the perforated plate (30).

In the computer-simulated experimental run, an electromagnetic field distribution locally varying between 92 V/m and 1387 V/m (not limiting) was achieved with only 60 mW (not limiting) of microwave power input. Considering that the fluid inevitably receives a turbulence when passing through the block (20), the exertion of electromagnetic field on bodies will remain at an extent in between these extrema. Indeed, a mean value of electromagnetic field throughout the block (20) is calculated to range between 700 V/m and 1000 V/m (not limiting). Thus, even with such low extent of power input, the electromagnetic field is very strong throughout the volume defined within the block (20).

Considering that the dielectric constant of water is higher than air, the wattage at microwave power input is clearly to be increased to achieve comparable extents of electromagnetic field distribution accordingly. Yet, the energy requirement of an apparatus (1) adapted accordingly, would still correspond to a low operational cost. Even with high extent of such percentage, the maximum local value of electromagnetic field in a parallel projection of a perforated plate (30) away from the block (20) is calculated as 1.8427 V/m. This result shows that the presence of a perforated plate (30) very effectively blocks the escape of microwaves away from the block (20). The microwave power input was based on the employment of two SMA WR90 adapters (30 mW each) as microwave emitting connectors (11) which are commercially available in the market at low cost. WR90 waveguides provide a single-mode provision of adjustable frequency within the range between 6.5 GHz and 13 GHz, and they have an even more acceptable accuracy within the range between 7 GHz and 12 GHz.

### Reference signs:

- 10: power source
- 11: microwave emitting connector
- 12: microwave receiving connector
- 13: circulator
- 14: signal generator
- 15: power supply
- 16: data management means
- 17: communication interface
- 18: fluid quality sensor
- 19: presence sensor
- 20: block
- 21: first side wall
- 22: second side wall
- 23: microwave reflector
- 30: perforated plate
- 60: port
- 61: pressurizing means
- 62: holding means
- 63: opening
- 100: apparatus
- C: corridor
- L: length
- W: width

## Claims

1. An apparatus (1) which includes a block (20) having a first side wall (21) and a second side wall (22) opposing said first side wall (21), arranged for allowing flow of fluids in an intended fluid flow direction (D) transverse to a plane passing through both the first side wall (21) and second side wall (22), and one or more microwave emitting connector(s) (11) arranged for provision of the block (20) with microwaves transverse to said intended fluid flow direction (D).

2. The apparatus (1) according to the claim 1, further including one or more microwave receiving connector(s) (12) arranged for collecting microwave beams released from the microwave emitting connector(s) (11) into the block (20), and one or more one or more circulator(s) (13) for at least partial recovery of the microwave energy from the microwave receiving connector (12) to supply the microwave emitting connector (11).

3. The apparatus (1) according to the claim 2, further including one or more power source(s) (10) arranged for replenishment of a difference between microwave energy extents at the microwave emitting connector (11) and the microwave receiving connector (12).

4. The apparatus according to any of the claims 1 to 3, wherein said one or more microwave emitting connectors (11) are arranged to scatter microwaves on a plane which is transverse to the intended fluid flow direction (D).

5. The apparatus according to any of the claims 1 to 4, wherein said one or more microwave emitting connectors (11) are provided with a motor-guided microwave directing means to allow setting and/or manipulation and/or alternation of directions of microwaves.

6. The apparatus according to any of the claims 1 to 5, provided with one or more reflectors (23) separating the block (20) into two or more corridors (C) for meandering of microwaves.

7. The apparatus according to the claim 6, wherein the block (20) is provided with a single microwave emitting connector (11) and with a single microwave receiving connector (12).

8. The apparatus (1) according to the claim 7, wherein said microwave emitting connector (11) and single microwave receiving connector (12) are provided at a single side of the block (20) in common.

9. The apparatus (1) according to any of the claims 1 to 7, provided with a plurality of perforated plates (30) arranged for sandwiching the block (20).

10. The apparatus (1) according to any of the claims 1 to 9, comprising a plurality of blocks (20) arranged in series.

11. The apparatus (1) according to the claim 10, wherein the plurality of blocks (20) is confined between perforated plates (30) arranged to allow said flow of the fluid through the blocks (20).

12. The apparatus (1) according to the claim 11, comprising one or more further perforated plates (30) which are sandwiched between consecutive blocks (20).

13. The apparatus (1) according to any of the claims 1 to 12, arranged for engaging with a fluid flow port (60).

14. The apparatus (1) according to the claim 13, further comprising a holding means (62) with fluid passage openings (63), the holding means having a size and shape to receive said one or more blocks (20), and preferably to attach said one or more blocks (20) to the port (60).

15. The apparatus (1) according to any of the claims 1 to 14, wherein said one or more microwave emitting connector(s) (11) are arranged for emitting a frequency within the range between 1 GHz and 20 GHz, preferably between 8.0 GHz and 9.5 GHz, more preferably between 8.2 GHz and 8.8 GHz, even more preferably between 8.3 GHz and 8.5 GHz.
